# EUROPEAN PATENT APPLICATION

(11) **EP 3 816 151 A1**
(43) Date of publication of application: **05.05.2021**
(21) Application number: 19826695.9
(22) Date of filing: 12.06.2019
(51) Int. Cl.: C07C 247/16, B01J 27/055, B01J 31/04, C07C 227/18, C07C 229/60, C07C 247/18, C07D 235/26, C07B 61/00

(54) **METHOD FOR PRODUCING ?-AZIDOANILINE DERIVATIVE OR ?, ?'-DIAZIDE DERIVATIVE**

(30) Priority: 28.06.2018 JP 2018122811
(71) Applicant: Tokuyama Corporation, Shunan-shi, Yamaguchi 745-8648 (JP)
(72) Inventor: SEKI Masahiko, Shunan-shi, Yamaguchi 745-8648 (JP)
(74) Representative: Dehns
(86) International application number: PCT/JP2019/023333
(87) International publication number: WO 2020/004043

(57) **Abstract**

Provided is a method for producing an α-azidoaniline derivative or an α,α'-diazide derivative using an aniline derivative as a starting material.

The present invention includes a method for producing an α-azidoaniline derivative or an α,α'-diazide derivative represented by Formula (2), including the step of: contacting an aniline derivative represented by Formula (1) with an azidating agent in presence of water, a persulfate, and a copper compound (In Formula (1), R¹ to R⁵ are each independently a hydrogen atom, a halogen atom, a nitro group, a cyano group, a substituted or unsubstituted alkyl group having 1 to 12 carbon atoms, a substituted or unsubstituted alkoxy group having 1 to 12 carbon atoms, a substituted or unsubstituted aralkyl group having 7 to 14 carbon atoms, a substituted or unsubstituted aryl group having 2 to 13 carbon atoms, a substituted or unsubstituted alkoxycarbonyl group having 2 to 7 carbon atoms, a substituted or unsubstituted carbonyl group having 1 to 20 carbon atoms, or optionally form an aliphatic hydrocarbon ring, an aromatic ring, or a heterocyclic ring together with an adjacent group. In Formula (2), R¹ to R³, and R⁵ have a meaning same as that of R¹ to R³, and R⁵ in Formula (1), and R⁴ is an azido group (N₃) or has a meaning same as that of R⁴ in Formula (1)).

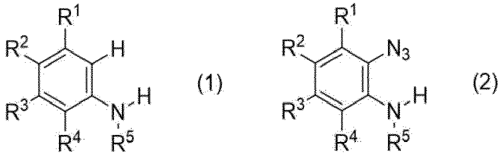

## Description

### Technical Field

The present invention relates to a novel method for producing an α-azidoaniline derivative or an α,α'-diazide derivative, which is useful as an intermediate for chemical products, active pharmaceutical ingredients (APIs) and the like.

### Background Art

A benzimidazole derivative is very useful as an intermediate for chemical products and APIs. For example, a benzimidazole derivative represented by Formula (7) below: 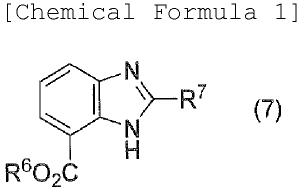 (wherein R⁶ is an alkyl group having 1 to 6 carbon atoms, an alkoxyalkyl group having 2 to 12 carbon atoms, an aralkyl group having 7 to 10 carbon atoms, or an alkoxyaralkyl group having 8 to 11 carbon atoms, and R⁷ is an alkyl group having 1 to 6 carbon atoms or an alkoxy group having 1 to 6 carbon atoms) is industrially very useful as an intermediate and the like for a sultan API such as candesartan cilexetil (for example, see Patent Literatures 1 to 3).

Usually, BIM, which is one of the benzimidazole derivatives represented by Formula (7) above, is synthesized by the following method.

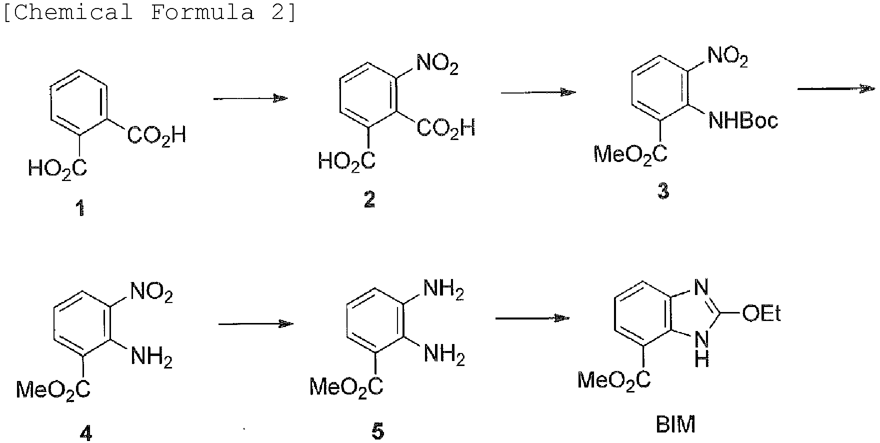

First, 2-amino-3-nitrobenzoic acid ester 4 is obtained from o-phthalic acid 1 through three steps. Then, the 2-amino-3-nitrobenzoic acid ester 4 is reduced to obtain diamino form 5. The diamino form 5 is further reduced to synthesize BIM.

However, this method is not always satisfactory as an industrial process because the method has many steps, and in addition, Curtius rearrangement in which an acyl azide, which is dangerous, is handled at high temperature (compounds 2 to 3) is employed. Further, when the amino group of the 2-amino-3-nitrobenzoic acid ester 4 is reduced, currently in the prior art, an expensive nickel catalyst (see Patent Literature 1) or palladium catalyst (see Patent Literature 2) is used, or a highly toxic tin compound (see Patent Literature 3) is used.

To produce an API such as candesartan cilexetil from a diaminobenzoic acid ester and a benzimidazole derivative represented by Formula (7) above, very many steps are subsequently required. Thus, these intermediates are desirably synthesized using a reagent that is as inexpensive as possible and easy to handle. The prior art has room for improvement in this regard.

Non Patent Literature 1 discloses azidation of an aniline derivative in which an inexpensive sodium azide and hydrogen peroxide are used in a water solvent using copper acetate as a catalyst.

### Citation List

### Patent Literature

Patent Literature 1: WO 2006/015134
Patent Literature 2: WO 2015/173970
Patent Literature 3: WO 2012/018325

### Non Patent Literature

Hongli Fang et.al, Journal of Organic Chemistry, 2017, 82, 20, 11212-11217

### Summary of Invention

### Technical Problem

In view of the above-mentioned present situation, an object of the present invention is to provide a method for more easily producing a diaminobenzoic acid ester and a benzimidazole derivative using less expensive materials.

### Solution to Problem

The present inventors have intensively studied to solve the above-mentioned problems. Eventually, they found that an azido group can be introduced into the carbon at α-position to the amino group of an aniline derivative by contacting an aniline derivative with an azidating agent in presence of water, a persulfate, and a copper compound. Thus, the present inventors applied the above-mentioned reaction to an anthranilic acid ester, which is inexpensive and readily available, and eventually, found that the carbon at α-position to the amino group of the anthranilic acid ester can be azidated, thereby completing the present invention.

That is, (1) the first present invention is a method for producing an α-azidoaniline derivative or an α,α'-diazide derivative represented by Formula (2) below: 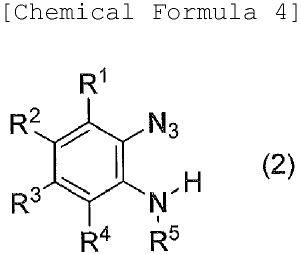 (wherein R¹ to R³, and R⁵ are each independently a hydrogen atom, a halogen atom, a nitro group, a cyano group, a substituted or unsubstituted alkyl group having 1 to 12 carbon atoms, a substituted or unsubstituted alkoxy group having 1 to 12 carbon atoms, a substituted or unsubstituted aralkyl group having 7 to 14 carbon atoms, a substituted or unsubstituted aryl group having 2 to 13 carbon atoms, a substituted or unsubstituted alkoxycarbonyl group having 2 to 7 carbon atoms, a substituted or unsubstituted carbonyl group having 1 to 20 carbon atoms, or optionally form an aliphatic hydrocarbon ring, an aromatic ring, or a heterocyclic ring together with an adjacent group, and R⁴ is an azido group (N₃), a hydrogen atom, a halogen atom, a nitro group, a cyano group, a substituted or unsubstituted alkyl group having 1 to 12 carbon atoms, a substituted or unsubstituted alkoxy group having 1 to 12 carbon atoms, a substituted or unsubstituted aralkyl group having 7 to 14 carbon atoms, a substituted or unsubstituted aryl group having 2 to 13 carbon atoms, a substituted or unsubstituted alkoxycarbonyl group having 2 to 7 carbon atoms, a substituted or unsubstituted carbonyl group having 1 to 20 carbon atoms, or optionally forms an aliphatic hydrocarbon ring, an aromatic ring, or a heterocyclic ring together with an adjacent group),
including the step of:
contacting an aniline derivative represented by Formula (1) below: 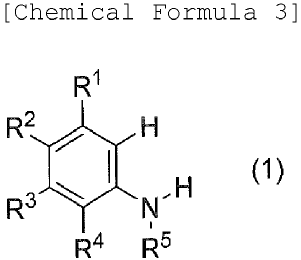 (wherein R¹ to R⁵ are each independently a hydrogen atom, a halogen atom, a nitro group, a cyano group, a substituted or unsubstituted alkyl group having 1 to 12 carbon atoms, a substituted or unsubstituted alkoxy group having 1 to 12 carbon atoms, a substituted or unsubstituted aralkyl group having 7 to 14 carbon atoms, a substituted or unsubstituted aryl group having 2 to 13 carbon atoms, a substituted or unsubstituted alkoxycarbonyl group having 2 to 7 carbon atoms, a substituted or unsubstituted carbonyl group having 1 to 20 carbon atoms, or optionally form an aliphatic hydrocarbon ring, an aromatic ring, or a heterocyclic ring together with an adjacent group)
with an azidating agent in presence of water, a persulfate, and a copper compound.

In the above-mentioned first present invention, the following aspects can be suitably employed:
(1a) The aniline derivative represented by Formula (1) above is an anthranilic acid ester represented by Formula (3) below: 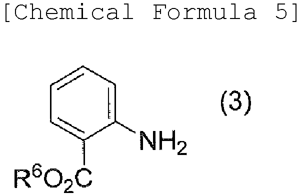 (wherein R⁶ is an alkyl group having 1 to 6 carbon atoms, an alkoxyalkyl group having 2 to 12 carbon atoms, an aralkyl group having 7 to 10 carbon atoms, or an alkoxyaralkyl group having 8 to 11 carbon atoms);
(1b) the azidating agent is at least one selected from sodium azide, tetra-n-butylammonium azide, tetramethylammonium azide, tetraethylammonium azide, and benzyltriethylammonium azide;
(1c) the persulfate is at least one selected from sodium persulfate, potassium persulfate, ammonium persulfate, and potassium hydrogen persulfate;
(1d) a reaction liquid when the aniline derivative is contacted with the azidating agent has a pH of 3.0 to 7.0; and
(1e) a reaction solvent used when the aniline derivative is contacted with the azidating agent is water or a water-containing solvent.
(2) The second present invention is a method for producing a diaminobenzoic acid ester represented by Formula (5) below: 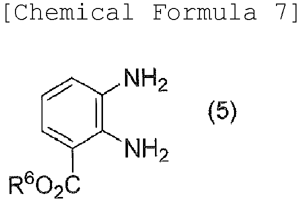 (wherein R⁶ is an alkyl group having 1 to 6 carbon atoms, an alkoxyalkyl group having 2 to 12 carbon atoms, an aralkyl group having 7 to 10 carbon atoms, or an alkoxyaralkyl group having 8 to 11 carbon atoms),
   including the step of:
   reducing an azido form of an anthranilic acid ester represented by Formula (4) below: 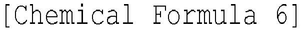 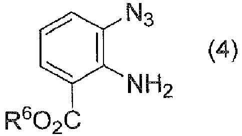 (wherein R⁶ has a meaning same as that of R⁶ in Formula (5) above).
   The second present invention preferably includes producing the azido form of an anthranilic acid ester of Formula (4) above by the method for production of the first present invention.
(3) The third present invention is a method for producing a benzimidazole derivative represented by Formula (7) below: 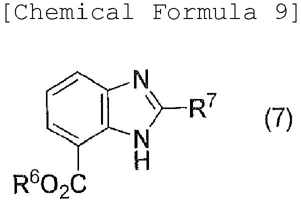 (wherein R⁶ has a meaning same as that of R⁶ in Formula (3) above, and R⁷ is an alkyl group having 1 to 6 carbon atoms or an alkoxy group having 1 to 6 carbon atoms),
   including the step of:
   producing a diaminobenzoic acid ester represented by Formula (5) above by the method for production of the second present invention, and then contacting the obtained diaminobenzoic acid ester with an ortho ester derivative represented by Formula (6) below: 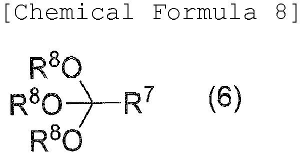 (wherein R⁷ has a meaning same as that of R⁷ in Formula (7) above, and R⁸ is an alkyl group having 1 to 6 carbon atoms, and is optionally same or different each other),
   in presence of an acid.
(4) The fourth present invention is an azido form of an anthranilic acid ester represented by Formula (4) below: 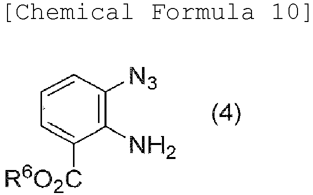 (wherein R⁶ is an alkyl group having 1 to 6 carbon atoms, an alkoxyalkyl group having 2 to 12 carbon atoms, an aralkyl group having 7 to 10 carbon atoms, or an alkoxyaralkyl group having 8 to 11 carbon atoms).

### Advantageous Effects of Invention

According to the present invention, an α-azidoaniline derivative or an α,α'-diazide derivative can be produced through fewer steps than in a conventional synthesis method using an inexpensive and readily available aniline derivative as a starting material, and in a safe method. The α-azidoaniline derivative or an α,α'-diazide derivative produced by the method for production of the present invention can be used as a raw material for synthesis of various compounds, and thus the industrial utility value of the present invention is very high.

In particular, by using an inexpensive and easily available anthranilic acid ester as a starting material and going through an α-azidoaniline derivative, a diaminobenzoic acid ester can be produced through fewer steps than in a conventional synthesis method. According to the above-mentioned method, a diaminobenzoic acid ester can be safely obtained without going through Curtius rearrangement in which a dangerous acyl azide is handled at high temperature.

By reacting the obtained diaminobenzoic acid ester with an ortho ester derivative, a benzimidazole derivative can be easily produced. The obtained benzimidazole derivative can be used as an intermediate for various chemical products and APIs such as candesartan cilexetil, and thus the industrial utility value of the present invention is very high.

### Description of Embodiments

The present invention is a method for producing an α-azidoaniline derivative or an α,α'-diazide derivative by contacting an aniline derivative represented by Formula (1) above with an azidating agent in presence of water, a persulfate, and a copper compound to introduce an azido group into the carbon at α-position to the amino group of the aniline derivative. An azido group is introduced into the aniline derivative by mixing an aniline derivative, an azidating agent, water, a persulfate, and a copper compound.

The process will be described step by step below.

### <Method for producing α-azidoaniline derivative or α,α'-diazide derivative>

### (Raw material compound)

### (Aniline derivative)

In the present invention, the aniline derivative used as a raw material is a compound represented by Formula (1) below:

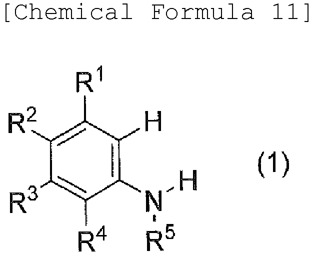

In Formula (1) above, R¹ to R⁵ are each independently a hydrogen atom, a substituted or unsubstituted alkyl group having 1 to 12 carbon atoms, a substituted or unsubstituted alkoxy group having 1 to 12 carbon atoms, a substituted or unsubstituted aralkyl group having 7 to 14 carbon atoms, a substituted or unsubstituted aryl group having 2 to 13 carbon atoms, or a substituted or unsubstituted alkoxycarbonyl group having 2 to 7 carbon atoms. R¹ to R⁵ may be a nitro group, a halogen group, a cyano group, or a substituted or unsubstituted carbonyl group having 1 to 20 carbon atoms. Examples of the substituent include an alkoxy group having 1 to 5 carbon atoms, a dialkylamino group having 2 to 10 carbon atoms, an acylamino group having 2 to 10 carbon atoms, an ester group having 2 to 10 carbon atoms, a nitro group, and a halogen group. The aralkyl group and the aryl group include, in addition to the aralkyl group and the aryl group having an aromatic hydrocarbon ring group as a ring structure, a heteroaralkyl group and a heteroaryl group having a heterocycle such as a furyl group, a thienyl group, and a triazolyl group.

R⁴ is also at the α-position to the amino group of the aniline derivative, and thus when R⁴ in Formula (1) above is a hydrogen atom, by performing the method for production of the present invention using such an aniline derivative as a raw material for synthesis, a predetermined amount of an α,α'-diazide derivative can be obtained in accordance with the amount of the raw material used.

R¹ to R⁵ in Formula (1) above may form an aliphatic hydrocarbon ring, an aromatic ring, or a heterocyclic ring together with an adjacent group, for example, R¹ and R², and R² and R³ may form such a ring.

The constituent atom that forms the ring may include, in addition to a carbon atom, a nitrogen atom, an oxygen atom, a sulfur atom, and a phosphorus atom. The number of the constituent atom that forms a ring (not including the number of carbon atoms in the benzene ring skeleton of the aniline derivative) is preferably 1 to 5 in view of industrial availability and the like.

From the viewpoint of availability as a raw material for candesartan cilexetil, the aniline derivative is particularly preferably an anthranilic acid ester represented by Formula (3) below:

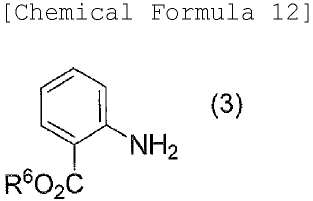

In Formula (3) above, R⁶ is an alkyl group having 1 to 6 carbon atoms, an alkoxyalkyl group having 2 to 12 carbon atoms, an aralkyl group having 7 to 10 carbon atoms, or an alkoxyaralkyl group having 8 to 11 carbon atoms. Among them, it is preferably an alkyl group having 1 to 5 carbon atoms for use as an intermediate for various substances and APIs. When R⁶ is an alkyl group having 1 to 5 carbon atoms, the compound can be suitably used as a raw material for candesartan cilexetil.

As the anthranilic acid ester represented by Formula (3), a commercially available one can be used. The anthranilic acid ester can be also produced by a known method.

### (Azidating agent)

In the present invention, an azido group is introduced into the aniline derivative represented by Formula (1) above by an azidating agent to produce an α-azidoaniline derivative or an α,α'-diazide derivative, an azido form of the aniline derivative represented by Formula (2) below: 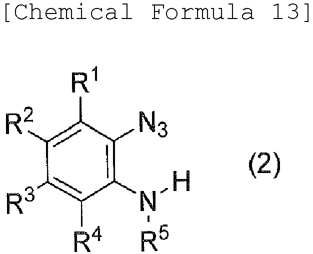 (wherein R¹ to R³, and R⁵ each have a meaning same as that of R¹ to R³, and R⁵ in Formula (1) above, and R⁴ is an azido group (N₃) or has a meaning same as that of R⁴ in Formula (1)).

As the azidating agent used in the present invention, a known azidating agent can be used without limitation.

Specific examples of the azidating agent include inorganic azides such as lithium azide (LiN₃), sodium azide (NaN₃), potassium azide (KN₃), cesium azide (CsN₃), and rubidium azide (RbN₃); and organic azides such as trimethylsilyl azide (TMSN₃), benzenesulfonyl azide (PhSO₂N₃), p-toluenesulfonyl azide (p-TolSO₂N₃), diphenylphosphoryl azide (DPPA), tetra-n-butylammonium azide, tetramethylammonium azide, tetraethylammonium azide, and benzyltriethylammonium azide.

Among the above-mentioned azidating agents, the azidating agent is at least one selected from sodium azide, tetra-n-butylammonium azide, tetramethylammonium azide, tetraethylammonium azide, and benzyltriethylammonium azide from the viewpoint of industrial availability, the yield, safety and price.

The amount of the azidating agent used is not particularly limited. To achieve the reduction in the amount used for safety improvement and cost-cutting, and a high conversion rate, 1 to 10 mol of an azidating agent is preferably used, 1 to 5 mol of an azidating agent is more preferably used, and 1 to 3 mol of an azidating agent is further preferably used per 1 mol of the aniline derivative.

In the present invention, the azidation by the azidating agent is performed in presence of water, a persulfate, and a copper compound.

### (Persulfate)

In the present invention, the persulfate to be present together with the azidating agent is not particularly limited. Examples of the persulfate include sodium persulfate, potassium persulfate, ammonium persulfate, potassium hydrogen persulfate, and oxone (peroxymonosulfate). Among these persulfates, the persulfate is preferably at least one selected from sodium persulfate, potassium persulfate, ammonium persulfate, and potassium hydrogen persulfate from the viewpoint of the yield, cost, and safety.

The amount of the persulfate used is not particularly limited. To achieve the oxidative regeneration of the copper compound as a catalyst and the proceeding of azidation such as oxidation of a benzene ring described below, 1 to 10 mol of the persulfate is preferably used, 1 to 4 mol of the persulfate is more preferably used, and 1 to 2 mol of the persulfate is further preferably used per 1 mol of the aniline derivative.

### (Copper compound)

In the present invention, the copper compound to be present together with the azidating agent is not particularly limited. Examples thereof include copper salts such as copper (I) chloride (CuCl), copper (II) chloride (CuCl₂), copper (I) bromide (CuBr), copper (II) bromide (CuBr₂), copper (I) iodide (CuI), copper (II) iodide (CuI₂), copper (I) acetate (CuOAc), copper (II) acetate (Cu(OAc)₂), copper (II) nitrate (Cu(NO₃)₂), and copper (II) sulfate (CuSO₄), copper oxides such as copper (I) oxide (Cu₂O) and copper (II) oxide (CuO), organic copper salts such as copper (II) trifluoromethanesulfonate (Cu(OTf)₂), copper (I) benzoate (CuOBz), and copper (II) benzoate (Cu(OBz)₂), and hydrates or solvates of these copper salts. Among them, from the viewpoint of the yield and cost, the copper compound is preferably copper (II) acetate or copper (II) sulfate, or a hydrate thereof.

The amount of the copper compound used is not particularly limited. To improve the conversion rate, 0.001 to 4 mol of the copper compound is preferably used, 0.001 to 2 mol of the copper compound is more preferably used, and 0.05 to 1 mol of the copper compound is further preferably used per 1 mol of the aniline derivative.

### (Water)

In the present invention, water to be present together with the azidating agent is used to dissolve the azidating agent and the copper compound and improve the reaction rate. The amount of water used is not particularly limited, and water may be used as a reaction solvent as described below. To improve the conversion rate of the aniline derivative to an α-azidoaniline derivative or an α,α'-diazide derivative, 1 to 1000 parts by mass of water is preferably used, 5 to 500 parts by mass of water is more preferably used, and 10 to 100 parts by mass of water is further preferably used per 1 part by mass of the aniline derivative. When an organic solvent described below is used, water can be used at a volume ratio of organic solvent/water of 0.1 to 10.

### (Reaction condition for producing α-azidoaniline derivative or an α,α'-diazide derivative)

### (Reaction solvent)

In the present invention, to produce an α-azidoaniline derivative or an α,α'-diazide derivative, the aniline derivative is contacted with an azidating agent in presence of water, a persulfate, and a copper compound. At that time, it is preferable that the aniline derivative and the azidating agent be mixed with stirring in a reaction solvent, and be sufficiently contacted each other. In the present invention, the reaction solvent used is not particularly limited as long as the reaction solvent does not adversely affect the aniline derivative, the azidating agent, the persulfate, and the copper compound, and the azido form of the aniline derivative can be smoothly produced. Considering the solubility, stability, reactivity, cost, safety and the like of the aniline derivative, the azidating agent, the persulfate, the copper compound, and the obtained α-azidoaniline derivative or an α,α'-diazide derivative, examples of the reaction solvent include water; acetate esters such as ethyl acetate, methyl acetate, butyl acetate, and isopropyl acetate; aliphatic nitriles such as acetonitrile and propionitrile; ethers such as tetrahydrofuran (THF), 2-methyltetrahydrofuran (2-methyl THF), 1,4-dioxane, t-butyl-methyl ether, dimethoxyethane, and diglyme; ketones such as acetone, diethyl ketone, and methyl ethyl ketone; halogenated hydrocarbons such as methylene chloride, chloroform, carbon tetrachloride, 1,2-dichloroethane, and chlorobenzene; aromatic hydrocarbons such as toluene and xylene; aliphatic hydrocarbons such as hexane and heptane; and aprotic polar solvents such as dimethyl sulfoxide (DMSO). These solvents can be also used as a mixed solvent or a water-containing solvent.

Among them, to increase the purity and the yield of the obtained α-azidoaniline derivative or an α,α'-diazide derivative, water, acetonitrile, ethyl acetate, toluene, methylene chloride, DMSO, or a mixed solvent or water-containing solvent thereof is preferably used.

In the present invention, when a reaction solvent is used, the reaction solvent is preferably used in an amount that allows all the components to be sufficiently mixed each other. Specifically, the reaction solvent is preferably used in an amount of 1 to 100 times, more preferably used in an amount of 1 to 50 times, and further preferably used in an amount of 2 to 30 times the volume of the aniline derivative. When a mixed solvent is used as the reaction solvent, the total amount of the mixed solvent may satisfy the above-mentioned range.

### (Method for introducing raw material into reaction system)

In the present invention, the method for introducing the aniline derivative, the azidating agent, water, the persulfate, the copper compound, and the reaction solvent into a reaction system (a place where the reaction is performed, such as a reaction vessel) is not particularly limited. That is, though the order of introduction is not limited, to promote the coordination of the copper salt to the amino group, it is preferred that the aniline derivative be placed in advance in the reaction solvent in the reaction system, the copper compound be introduced thereto, the mixture be stirred and mixed, then the azidating agent be added, the mixture be stirred and mixed, and then the persulfate be introduced. The azidating agent and the persulfate can be added simultaneously after the aniline derivative is placed in advance in the reaction solvent in the reaction system, the copper compound is introduced thereto, and the mixture is stirred and mixed. The persulfate is preferably dissolved in water and introduced into the system as needed.

In the above-mentioned addition method, the time from the introduction of the copper compound to the addition of the azidating agent is preferably 0 to 2 hours, more preferably 5 minutes to 1 hour. The time from the addition of the azidating agent to the introduction of the persulfate is preferably 0 to 2 hours, more preferably 5 minutes to 1 hour. Further, the persulfate is preferably added over 0 to 10 hours, and more preferably added over 10 minutes to 5 hours.

The pH of the reaction liquid during the reaction is preferably pH 3.0 to 7.0, more preferably pH 3.5 to 6.0, and further preferably pH 3.5 to 5.0 from the viewpoint of, for example, the activity of the persulfate or the azidating agent, and the stability of the aniline derivative as a raw material and the azide compound as a product. Though the pH can be in the above-mentioned range before the addition of the persulfate, the reaction can be performed while checking the pH according to the proceeding of the reaction and adding an aqueous alkali solution to adjust the pH to fall within the above-mentioned range because the pH becomes more acidic as the reaction proceeds after the addition of the persulfate.

### (Reaction temperature)

The reaction temperature can be appropriately determined depending on the solvent used. Specifically, the temperature is preferably -30°C or more and the reflux temperature or less of the reaction solvent containing the aniline derivative, the azidating agent, the persulfate, and the copper compound. More specifically, the temperature is preferably -30°C or more and 100°C or less, more preferably 10°C or more and 80°C or less, and particularly preferably 20°C or more and 60°C or less. By performing the reaction at the above-mentioned temperature, the reaction is promoted, the decomposition of the product is suppressed, and an α-azidoaniline derivative or an α,α'-diazide derivative having a higher purity can be obtained in a higher yield.

### (Other conditions)

In the present invention, the following conditions are preferably employed as other reaction conditions. The reaction time can be appropriately determined depending on the consumption of the aniline derivative, the production amount of the α-azidoaniline derivative or an α,α'-diazide derivative, the scale of the reaction and the like. The reaction time can be usually 10 minutes or more and 48 hours or less, and is preferably 1 hour or more and 24 hours or less.

In the present invention, the conditions of the reaction atmosphere are not particularly limited, and can be any of an air atmosphere, an inert gas atmosphere, and a hydrogen atmosphere. Among them, an inert atmosphere of nitrogen or the like is preferable considering, for example, the suppression of mixing of moisture.

The reaction system can be under atmospheric pressure, under pressure, or under reduced pressure. Among them, the reaction is preferably performed under atmospheric pressure.

By performing the reaction under the above-mentioned conditions, the α-azidoaniline derivative or an α,α'-diazide derivative can be obtained.

### (Method for purifying and taking out α-azidoaniline derivative or α,α'-diazide derivative)

The α-azidoaniline derivative or an α,α'-diazide derivative obtained under the above-mentioned reaction conditions is preferably taken out of the reaction system by the following method. Specifically, the α-azidoaniline derivative or an α,α'-diazide derivative is preferably extracted with a poorly water-soluble organic solvent such as ethyl acetate, chloroform, and methylene chloride by contacting the obtained reaction liquid with the poorly water-soluble organic solvent. Then, the poorly water-soluble organic solvent containing the α-azidoaniline derivative or an α,α'-diazide derivative is preferably washed with water to remove copper salts and inorganic salts.

The obtained α-azidoaniline derivative or an α,α'-diazide derivative can be further purified by a known method such as column separation and recrystallization.

### <Method for producing diaminobenzoic acid ester from azido form of anthranilic acid ester>

In the present invention, a diaminobenzoic acid ester can be produced by reducing an azido form of an anthranilic acid ester by the method described below.

When an anthranilic acid ester is used as an aniline derivative in <Method for producing α-azidoaniline derivative or α,α'-diazide derivative> above, an azido form of an anthranilic acid ester is obtained. A diaminobenzoic acid ester can be produced by reducing the azido form of an anthranilic acid ester. For the reduction of the azido form of an anthranilic acid ester, a known reduction method can be employed without particular limitation. Specifically, an azido form of an anthranilic acid ester can be reduced by, for example, contacting an azido form of an anthranilic acid ester with hydrogen, formic acid or ammonium formate, or zinc dust, or contacting an azido form of an anthranilic acid ester with phosphine to form an iminophosphorane form of an anthranilic acid ester, and then hydrolyzing the iminophosphorane form.

### (Method for reducing azido form using hydrogen)

In the present invention, examples of the method for contacting an azido form of an anthranilic acid ester with hydrogen to reduce the azido form of an anthranilic acid ester include a method of contacting an azido form of an anthranilic acid ester with hydrogen gas in presence of a catalyst such as a palladium catalyst such as palladium carbon and palladium black, and a nickel catalyst such as Raney nickel.

The hydrogen gas can be supplied to the solvent of the azido form of anthranilic acid ester in a pressure range of 1 atm to 100 atm, preferably 1 to 80 atm, more preferably 1 to 50 atm.

The amount of the catalyst used is not particularly limited, and is preferably 0.0001 to 10 mol, more preferably 0.0005 to 5 mol, and further preferably 0.001 to 2 mol in terms of metal atoms in the catalyst per 1 mol of an azido form of an anthranilic acid ester.

In the present invention, to contact an azido form of an anthranilic acid ester with hydrogen for the reduction, they are preferably mixed with stirring. When they are mixed with stirring, reduction is preferably performed in a solvent. The solvent used is not particularly limited as long as it does not affect the reduction reaction. Specific examples thereof include alcohols such as methanol, ethanol, and isopropanol, 1,4-dioxane, 1,2-dimethoxyethane (DME), THF, and water or a mixed solvent of water and the above-mentioned solvent.

The temperature at which the reduction reaction is performed is not particularly limited, and is preferably 0 to 120°C, further preferably 20 to 100°C. The reaction time is also not particularly limited, and can be appropriately determined according to the conversion rate of an azido form of an anthranilic acid ester to a diaminobenzoic acid ester. The atmosphere is also not particularly limited, and can be any of an air atmosphere and an inert gas atmosphere. Among them, considering the operability and the like, an air atmosphere is preferable.

### (Method for reducing azido form using formic acid or ammonium formate)

In the present invention, when the reduction is performing by contacting an azido form of an anthranilic acid ester with formic acid or ammonium formate, the amount of formic acid or ammonium formate used is not particularly limited, and is preferably 1 to 100 mol, more preferably 1 to 50 mol, further preferably 1 to 10 mol per 1 mol of an azido form of an anthranilic acid ester.

To contact an azido form of an anthranilic acid ester with formic acid or ammonium formate, they are preferably mixed with stirring. When they are mixed with stirring, they are preferably contacted in a solvent. The solvent used is not particularly limited as long as it does not affect the reduction reaction. Specific examples thereof include alcohols such as methanol, ethanol, and isopropanol, 1,4-dioxane, 1,2-dimethoxyethane (DME), THF, and water or a mixed solvent of water and the above-mentioned solvent.

The temperature at which the reduction reaction is performed is not particularly limited, and is preferably 0 to 120°C, further preferably 20 to 100°C. The reaction time is also not particularly limited, and can be appropriately determined according to the conversion rate of an azido form of an anthranilic acid ester to a diaminobenzoic acid ester. The atmosphere is also not particularly limited, and can be any of an air atmosphere and an inert gas atmosphere. Among them, considering the operability and the like, an air atmosphere is preferable.

### (Method for reducing azido form using zinc)

In the present invention, when the reduction is performed by contacting an azido form of an anthranilic acid ester with zinc dust, the azido form of an anthranilic acid ester is contacted with zinc dust in presence of an acidic substance such as ammonium chloride, hydrochloric acid, and sulfuric acid. Ammonium chloride is preferred as the acidic substance.

The amount of the zinc dust used is not particularly limited, and is preferably 1 to 10 mol, more preferably 1 to 8 mol, and further preferably 1 to 5 mol per 1 mol of an azido form of an anthranilic acid ester. When ammonium chloride is used as the acidic substance, the amount of ammonium chloride used is preferably 1 to 10 mol per 1 mol of zinc dust.

Similarly to the case of reduction using hydrogen, an azido form of an anthranilic acid ester, zinc dust, and an acidic substance are preferably mixed with stirring in a solvent. The solvent used is not particularly limited as long as it does not affect the reduction reaction. Specific examples thereof include ethyl acetate, methyl acetate, butyl acetate, isopropyl acetate, acetonitrile, propionitrile, methanol, ethanol, propanol, isopropanol, butanol, 2-butanol, tert-butanol, THF, 2-methyl THF, 1,4-dioxane, t-butyl-methyl ether, dimethoxyethane, diglyme, acetone, diethyl ketone, methyl ethyl ketone, methylene chloride, chloroform, carbon tetrachloride, 1,2-dichloroethane, chlorobenzene, toluene, DMSO, water, and a mixed solvent of these solvents. Among them, a mixed solvent of ethanol and water is preferably used from the viewpoint of reaction acceleration, cost, safety and the like.

The solvent is preferably used in an amount of 1 to 200 times, more preferably used in an amount of 1 to 100 times, and further preferably used in an amount of 1 to 50 times against the volume of the azido form of an anthranilic acid ester.

The method for introducing an azido form of an anthranilic acid ester, zinc dust, an acidic substance, and a reaction solvent into a reaction system (a place where the reaction is performed, such as a reaction vessel) is not particularly limited. That is, the order of introduction is not limited. For example, an azido form of an anthranilic acid ester and an acidic substance are placed in advance in a reaction system, a solvent is added thereto and dissolved, and then zinc dust is added to start the reduction reaction.

The temperature at which the reduction reaction is performed using zinc dust is not particularly limited, and is preferably 0 to 150°C, further preferably 10 to 100°C. The reaction time is also not particularly limited, and is preferably 0.5 to 24 hours, further preferably 1 to 17 hours. The atmosphere is also not particularly limited, and can be any of an air atmosphere and an inert gas atmosphere. Among them, considering the operability and the like, an air atmosphere is preferable.

After completion of the reaction, a diaminobenzoic acid ester can be obtained by separating residues by filtration, and washing the filtrate with water.

### (Method for reducing azido form using phosphine)

In the present invention, the reduction reaction of an azido form of an anthranilic acid ester to a diaminobenzoic acid ester is performed by contacting the azido form of an anthranilic acid ester with phosphine to obtain an iminophosphorane form of an anthranilic acid ester, and then hydrolyzing the iminophosphorane form.

Examples of the phosphine include an aromatic phosphine such as triphenylphosphine (PPh₃) and an alkylphosphine such as tributylphosphine. From the viewpoint of reactivity, cost, and safety, triphenylphosphine is preferably used.

The amount of the triphenylphosphine used is not particularly limited, and is preferably 1 to 10 mol, more preferably 1 to 8 mol, and further preferably 1 to 5 mol per 1 mol of an azido form of an anthranilic acid ester.

To contact an azido form of an anthranilic acid ester with phosphine, they are preferably mixed with stirring in a solvent. The solvent used is not particularly limited as long as it does not affect the reduction reaction. Specific examples thereof include ethyl acetate, methyl acetate, butyl acetate, isopropyl acetate, acetonitrile, propionitrile, THF, 2-methyl THF, 1,4-dioxane, t-butyl-methyl ether, dimethoxyethane, diglyme, acetone, diethyl ketone, methyl ethyl ketone, methylene chloride, chloroform, carbon tetrachloride, 1,2-dichloroethane, chlorobenzene, toluene, xylene, hexane, heptane, DMSO, water, and a mixed solvent of these solvents. Among them, water or a mixed solvent of THF and water is preferably used from the viewpoint of the yield, cost, and safety.

The solvent is preferably used in an amount of 1 to 200 times, more preferably used in an amount of 1 to 100 times, and further preferably used in an amount of 1 to 50 times against the volume of the azido form of an anthranilic acid ester.

The temperature at which the reduction reaction is performed is not particularly limited, and is preferably 0 to 120°C, further preferably 10 to 100°C. The reaction time is also not particularly limited, and is preferably 0.1 to 48 hours, further preferably 1 to 24 hours. The atmosphere is also not particularly limited, and can be any of an air atmosphere and an inert gas atmosphere. Among them, considering the operability and the like, an air atmosphere is preferable.

Then, a diaminobenzoic acid ester is synthesized by hydrolyzing the iminophosphorane form obtained by the reaction between the azido form of an anthranilic acid ester and phosphine. The obtained iminophosphorane form can be separated and hydrolyzed, or can be hydrolyzed in a reaction liquid without being separated.

The iminophosphorane form can be hydrolyzed by contacting the iminophosphorane form with an acid.

The acid used for the hydrolysis is not particularly limited, and examples thereof include known acids. From the viewpoint of the yield, cost, and safety, hydrochloric acid, sulfuric acid, methanesulfonic acid, p-toluenesulfonic acid, and phosphoric acid are preferred, and hydrochloric acid is more preferred.

The amount of the acid added is not particularly limited, and the iminophosphorane form is preferably hydrolyzed in a reaction liquid having pH of 4 or less.

To contact the iminophosphorane form with an acid for hydrolysis, they are preferably mixed with stirring in a solvent. When the iminophosphorane form is hydrolyzed following the reaction between an azido form of an anthranilic acid ester and phosphine without separation, an acid can be added to the solution after the reaction. When the iminophosphorane form is separated and hydrolyzed, as the solvent used, the same solvent as the solvent used for the reaction between the azido form of an anthranilic acid ester and phosphine can be used.

Water needs to be present during the hydrolysis, and water or a mixed solvent of water and another solvent is used as the solvent. When hydrolysis is performed following the reaction between the azido form of an anthranilic acid ester and phosphine, and water is not contained in the solvent, water can be added separately during the hydrolysis. The amount of water added is not particularly limited, and is preferably 0.01 to 200 times the volume of the iminophosphorane form.

The temperature at which the hydrolysis is performed is not particularly limited, and is preferably 10 to 150°C, further preferably 20 to 120°C. The reaction time is also not particularly limited, and is preferably 0.5 to 48 hours, further preferably 1 to 24 hours. The atmosphere is also not particularly limited, and can be any of an air atmosphere and an inert gas atmosphere. Among them, considering the operability and the like, an air atmosphere is preferable.

A diaminobenzoic acid ester can be obtained by alkalifying the reaction liquid after completion of the hydrolysis with a base, and then subjecting it to solvent extraction and the like.

The diaminobenzoic acid ester obtained by reducing the azido form of an anthranilic acid ester can be further purified by a known method such as recrystallization and column separation.

### <Method for producing benzimidazole derivative from diaminobenzoic acid ester>

A method for producing a benzimidazole derivative by contacting the diaminobenzoic acid ester obtained above with an ortho ester derivative in presence of an acid will be described. The synthesis reaction itself of a benzimidazole derivative by contacting a diaminobenzoic acid ester with an ortho ester derivative is known, and the method described in Patent Literature 1 or the like can be employed.

### (Ortho ester derivative)

In the present invention, the ortho ester derivative to be reacted with the diaminobenzoic acid ester is represented by Formula (6) below:

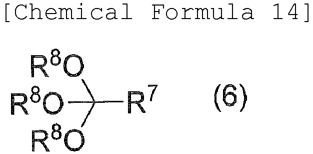

In Formula above, R⁷ is an alkyl group having 1 to 6 carbon atoms or an alkoxy group having 1 to 6 carbon atoms. When R⁷ is an alkoxy group, the ortho ester derivative is a compound having four alkoxy groups. Among R⁷, to use the obtained benzimidazole derivative as an intermediate of candesartan cilexetil, an ethoxy group is preferable. R⁸ is an alkyl group having 1 to 6 carbon atoms, and is optionally same or different each other. As such an ortho ester derivative, a commercially available one can be used.

In the reaction between the diaminobenzoic acid ester and the ortho ester derivative, the amount of the ortho ester derivative used is not particularly limited, and is preferably 0.5 to 10 mol, further preferably 0.95 to 2 mol per 1 mol of the diaminobenzoic acid ester.

### (Acid)

The reaction between the diaminobenzoic acid ester and the ortho ester derivative is performed in presence of an acid. The acid is not particularly limited, and inorganic acids such as hydrochloric acid and sulfuric acid, and organic acids such as formic acid, acetic acid, methanesulfonic acid, and p-toluenesulfonic acid can be used. Among them, organic acids such as acetic acid are preferably used from the viewpoint of handleability. At this time, the acid used can be also used as a reaction solvent.

The amount of the acid used is not particularly limited, and can be added in an excess amount when the acid is used as a reaction solvent. However, considering the post-treatment after the reaction and the like, 0.5 to 10 mL of an acid is preferably used, and 0.5 to 5 mL of an acid is more preferably used per 1 g of the diaminobenzoic acid ester. When an organic solvent is used as a reaction solvent, considering the reaction proceeding, post-treatment and the like, 0.1 to 10 mol of an acid is preferably used, and 0.5 to 3 mol of an acid is further preferably used per 1 mol of the diaminobenzoic acid ester.

### (Production conditions of benzimidazole derivative)

In the method for producing a benzimidazole derivative of the present invention, the diaminobenzoic acid ester is contacted with the ortho ester derivative in presence of an acid.

When an organic solvent is used as a reaction solvent, the organic solvent is not particularly limited as long as it does not adversely affect the diaminobenzoic acid ester, the acid, and the ortho ester derivative. However, to increase the reaction temperature, shorten the reaction time, and facilitate the post-treatment, the reaction solvent is preferably ethyl acetate, toluene, tetrahydrofuran (THF) and the like, and more preferably toluene.

In the reaction between the diaminobenzoic acid ester and the ortho ester derivative, a condensation reaction between an amino group and an alkoxy group occurs, then a cyclization reaction occurs, and a benzimidazole derivative represented by Formula (7) below is obtained: 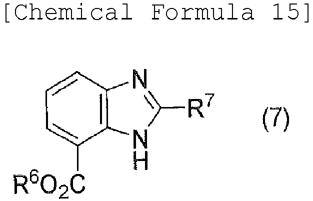 (wherein R⁶ has a meaning same as that of R⁶ in Formula (3) above, and R⁷ has a meaning same as that of R⁷ in Formula (6) above)

In this reaction, the reaction temperature can be appropriately determined depending on the set reaction conditions, and is preferably 0 to 150°C, more preferably 10 to 100°C, and particularly preferably 10 to 50°C. When the reaction temperature satisfies the above-mentioned range, the amount of impurities produced as by-products in the reaction can be reduced. The reaction time of 0.5 to 5 hours is sufficient. The atmosphere is not particularly limited, and can be an air atmosphere. Further, the reaction can proceed under any of reduced pressure, increased pressure, and atmospheric pressure.

The obtained benzimidazole derivative can be taken out of the reaction system by a known method. The benzimidazole derivative can be purified by a known method.

The obtained benzimidazole derivative can be suitably used as an intermediate (a raw material) of a sultan API such as candesartan cilexetil.

### <Azido form of anthranilic acid ester>

The azido form of an anthranilic acid ester of the present invention is represented by Formula (4) below: 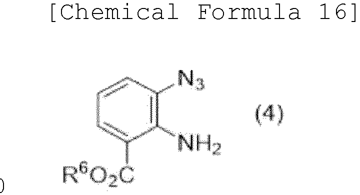 (wherein R⁶ has a meaning same as that of R⁶ in Formula (3) above). The azido form of an anthranilic acid ester can be suitably used as an intermediate (a raw material) when the benzimidazole derivative is produced.

The azido form of an anthranilic acid ester can be produced, for example, by an operation same as that in the case of using an anthranilic acid ester represented by Formula (3) below as an aniline derivative: 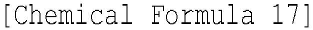 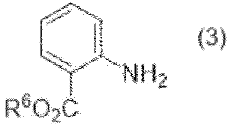 (wherein R⁶ has a meaning same as that of R⁶ in Formula (3) above), in <Method for producing α-azidoaniline derivative or α,α'-diazide derivative> above.

### Examples

Hereinafter, though the present invention will be described in detail with reference to Examples, Examples are specific examples and the present invention is not limited thereto.

The purity evaluation in Examples was performed by the following method using high performance liquid chromatography (HPLC).

### <HPLC measurement conditions>

Instrument: High performance liquid chromatography (HPLC)
Detector: Ultraviolet absorption photometer (measurement wavelength: 254 nm)
Column: XBridge C18 having an inner diameter of 4.6 mm and a length of 15 cm (a particle diameter of 5 µm)

### (manufactured by Waters Corporation)

Column temperature: constant at 30°C
Sample temperature: constant at 25°C
Flow rate: 1.0 mL/min
Measurement time: 20 minutes
Mobile phase A: acetonitrile
Mobile phase B: water
Delivery of mobile phase: concentration gradient control is performed by changing the mixing ratio of mobile phase A to mobile phase B as follows according to the measurement target.

### Measurement target: methyl 2,3-diaminobenzoate methyl 2-ethoxy-1H-benzimidazole-7-carboxylate

### Mixing ratio of mobile phase A to mobile phase B: 20 to 100% acetonitrile (0 to 20 min)

Under the above-mentioned conditions, the peak of methyl 2,3-diaminobenzoate (the diaminobenzoic acid ester) is observed at about 5.9 minutes, and the peak of methyl 2-ethoxy-1H-benzimidazole-7-carboxylate (the benzimidazole derivative) is observed at about 7.3 minutes.

### Measurement target: methyl anthranilate 3-azidoanthranilic acid ester

### Mixing ratio of mobile phase A to mobile phase B: 50 to 100% acetonitrile (0 to 20 min)

Under the above-mentioned conditions, the peak of methyl anthranilate (the anthranilic acid ester) is observed at about 3.7 minutes and the peak of 3-azidoanthranilic acid ester (the azido form of an anthranilic acid ester) is observed at about 5.8 minutes.

In the Examples below, the purities of the anthranilic acid ester, the azido form of an anthranilic acid ester, the diaminobenzoic acid ester, and the benzimidazole derivative are all ratios of the peak area value of each compound to the total of all peak area values (excluding solvent-derived peaks) measured under the above-mentioned conditions.

In Examples 1 to 15, the synthesis of methyl 3-azidoanthranilate (simply referred to as "synthesis" in Examples 1 to 15) was performed by the azidation shown in Formula below.

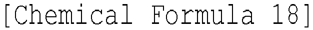

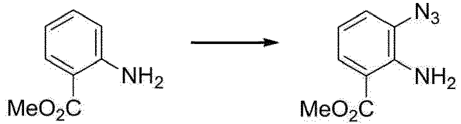

### [Example 1] (Synthesis in which copper (II) acetate monohydrate is used)

To copper (II) acetate monohydrate (0.17 g, 0.85 mmol), water (10 mL) was added to dissolve the copper (II) acetate monohydrate at 25°C. To this solution, methyl anthranilate (0.5 g, 3.30 mmol) and acetonitrile (20 mL) were added, and the mixture was stirred at 40°C for 10 minutes. Sodium azide (0.43 g, 6.61 mmol) was added thereto and the mixture was stirred for 10 minutes. Acetic acid (about 1 mL) was added thereto to adjust the pH of the reaction liquid to 5.8, and then the mixture was stirred at 40°C for 5 minutes. To this mixture, sodium persulfate (1.18 g, 4.96 mmol) was added at a time at the same temperature, and the mixture was stirred for 3 hours. During this time, the pH of the reaction liquid gradually became acidic. When the pH reached about 4.0, 24% aqueous sodium hydroxide was added to maintain the pH at 4.0 to 4.5.

After completion of the reaction, the reaction liquid was cooled to 15°C or less, sodium thiosulfate was added until the color of the iodine starch paper disappeared, and then the pH was adjusted to 0.5 with concentrated hydrochloric acid. HPLC analysis of the organic layer and the aqueous layer confirmed that the reaction liquid contained methyl 3-azidoanthranilate (469 mg, 73.9%).

Ethyl acetate (200 mL) was added to the reaction liquid, and the mixture was filtered. The filtrate was concentrated and then purified through a silica gel column (hexane/ethyl acetate = 20 : 1) to obtain methyl 3-azidoanthranilate as crystals (446 mg, 70.2%). The analytical values of methyl 3-azidoanthranilate were as follows.
Melting point: 61 to 63°C
IR (KBr): 2116, 1685 cm⁻¹
¹H-NMR (CDCl₃) : δ 3.85 (s, 3H), 6.00 (brs, 2H), 6.5 to 6.90 (m, 1H), 7.00 to 7.40 (m, 1H), 7.60 to 7.90 (m, 1H)
¹³C-NMR (CDCl₃): 168.2, 142.6, 127.6, 125.9, 122.0, 115.5, 111.4, 51.8

### [Example 2] (Synthesis in which copper (II) sulfate pentahydrate is used)

To copper (II) sulfate pentahydrate (0.21 g, 0.84 mmol), water (8 mL) was added to dissolve the copper (II) sulfate pentahydrate at room temperature. To this solution, methyl anthranilate (0.5 g, 3.30 mmol) and acetonitrile (20 mL) were added, and the mixture was stirred at 40°C for 10 minutes. Sodium azide (0.43 g, 6.61 mmol) was added to this solution at 40°C and the mixture was stirred for 10 minutes. To this reaction liquid, 20% sulfuric acid was added at 40°C to adjust the pH of the reaction liquid to 5.0, and the mixture was stirred at the same temperature for 5 minutes. To this reaction liquid, a solution obtained by dissolving sodium persulfate (1.18 g, 4.96 mmol) in water (3 mL) was dropped at 40°C while adjusting the pH of the reaction liquid to 4.0 to 5.0 with 24% aqueous sodium hydroxide.

After completion of dropping, the reaction liquid was stirred for 1 hour. The reaction liquid was cooled to 15°C or less, sodium thiosulfate was added until the color of the iodine starch paper disappeared, and then the pH was adjusted to 0.5 by addition of concentrated hydrochloric acid. HPLC analysis of the obtained liquid mixture confirmed that the liquid mixture contained methyl 3-azidoanthranilate (451 mg, 71.1%).

### [Example 3] (Synthesis in which azidation is performed without adjusting pH during azidation)

The same placement and reaction treatment as in Example 1 were performed except that water (5 mL) and acetonitrile (5 mL) were used as solvents, and the reaction was performed at 25°C for 17 hours without adjusting the pH to a predetermined range after the pH of the reaction liquid before addition of sodium persulfate was adjusted to 5.8. As a result of HPLC analysis of the treated solution, it was confirmed that the reaction liquid contained methyl 3-azidoanthranilate (178 mg, 28.2%).

### [Example 4] (Synthesis in which ammonium persulfate is used)

The same placement and post-treatment as in Example 3 were performed except that ammonium persulfate was used instead of sodium persulfate. As a result of HPLC analysis of the treated solution, it was confirmed that the reaction liquid contained methyl 3-azidoanthranilate (143 mg, 22.6%).

### [Example 5] (Synthesis in which reaction is performed in reaction liquid having pH of 3.0 to 3.5)

In the procedure of Example 1, acetonitrile (10 mL) and water (5 mL) were used as solvents, and the pH of the reaction liquid was maintained at 3.0 to 3.5 by adding 24% aqueous sodium hydroxide. Except the above, the same preparation and reaction treatment as in Example 1 were performed. As a result of HPLC analysis of the treated solution, it was confirmed that the reaction liquid contained methyl 3-azidoanthranilate (335 mg, 52.9%).

### [Example 6] (Synthesis in which reaction is performed in reaction liquid having pH of 3.5 to 4.0)

In the procedure of Example 1, acetonitrile (10 mL) and water (5 mL) were used as solvents, and the pH of the reaction liquid was maintained at 3.5 to 4.0 by adding 24% aqueous sodium hydroxide. Except the above, the same preparation and reaction treatment as in Example 1 were performed. As a result of HPLC analysis of the treated solution, it was confirmed that the reaction liquid contained methyl 3-azidoanthranilate (417 mg, 65.8%).

### [Example 7] (Synthesis in which reaction is performed in reaction liquid having pH of 4.0 to 5.0)

In the procedure of Example 1, acetonitrile (10 mL) and water (5 mL) were used as solvents, and the pH of the reaction liquid was maintained at 4.0 to 5.0 by adding 24% aqueous sodium hydroxide. Except the above, the same preparation and reaction treatment as in Example 1 were performed. As a result of HPLC analysis of the treated solution, it was confirmed that the reaction liquid contained methyl 3-azidoanthranilate (414 mg, 65.3%).

### [Example 8] (Synthesis in which reaction is performed in reaction liquid having pH of 5.0 to 6.0)

In the procedure of Example 1, acetonitrile (10 mL) and water (5 mL) were used as solvents, and the pH of the reaction liquid was maintained at 5.0 to 6.0 by adding 24% aqueous sodium hydroxide. Except the above, the same preparation and reaction treatment as in Example 1 were performed. As a result of HPLC analysis of the treated solution, it was confirmed that the reaction liquid contained methyl 3-azidoanthranilate (370 mg, 58.4%).

### [Example 9] (Synthesis in which reaction is performed in reaction liquid having pH of 6.0 to 7.0)

In the procedure of Example 1, acetonitrile (10 mL) and water (5 mL) were used as solvents, and the pH of the reaction liquid was maintained at 6.0 to 7.0 by adding 24% aqueous sodium hydroxide. Except the above, the same preparation and reaction treatment as in Example 1 were performed. As a result of HPLC analysis of the treated solution, it was confirmed that the reaction liquid contained methyl 3-azidoanthranilate (319 mg, 50.4%).

### [Example 10] (Synthesis in which amount of copper (II) acetate hydrate is increased and azidation is performed at 20°C for 17 hours)

The same placement and reaction treatment as in Example 1 were performed except that copper (II) acetate monohydrate (0.60 g, 3.31 mmol) was added, and the reaction was performed at 20°C for 17 hours. As a result of HPLC analysis of the treated solution, it was confirmed that the reaction liquid contained methyl 3-azidoanthranilate (419 mg, 66.0%).

### [Example 11] (Synthesis in which azidation is performed at a reaction temperature of 20°C for 17 hours)

The same placement and reaction treatment as in Example 1 were performed except that the reaction was performed at 20°C for 17 hours. As a result of HPLC analysis of the treated solution, it was confirmed that the reaction liquid contained methyl 3-azidoanthranilate (178 mg, 28.0%).

### [Example 12] (Synthesis in which azidation is performed for 4 hours)

The same placement and reaction treatment as in Example 1 were performed except that the reaction was performed for 4 hours. As a result of HPLC analysis of the treated solution, it was confirmed that the reaction liquid contained methyl 3-azidoanthranilate (413 mg, 65.0%).

### [Example 13] (Synthesis in which copper (II) sulfate pentahydrate is used and azidation is performed for 3 hours)

The same placement and reaction treatment as in Example 2 were performed except that the reaction was performed for 3 hours. As a result of HPLC analysis of the treated solution, it was confirmed that the reaction liquid contained methyl 3-azidoanthranilate (451 mg, 71.0%).

### [Example 14] (Synthesis in which copper (II) sulfate pentahydrate is used, the pH of the reaction liquid is adjusted to 4.5 after addition of azidating agent, and then sodium persulfate is added to perform azidation for 3 hours)

The same placement and reaction treatment as in Example 2 were performed except that the pH after addition of sodium azide, that is, the pH at the time of addition of sodium persulfate was adjusted to 4.5, and then an aqueous solution of sodium persulfate was added at once to perform reaction for 3 hours. As a result of HPLC analysis of the treated solution, it was confirmed that the reaction liquid contained methyl 3-azidoanthranilate (508 mg, 80.0%).

### [Example 15] (Synthesis in which copper (II) sulfate pentahydrate is used, the pH of the reaction liquid is adjusted to 4.5, and then sodium persulfate and sodium azide are added at once)

To copper (II) sulfate pentahydrate (0.41 g, 1.62 mmol, 25 mol%), water (5 mL) and acetonitrile (10 ml) were added to dissolve the copper (II) sulfate pentahydrate at room temperature. To this solution, methyl anthranilate (1.0 g, 6.60 mmol) was added, and the mixture was stirred at 40°C for 15 minutes. To this solution, 24% aqueous sodium hydroxide was added at 40°C to adjust the pH of the reaction liquid to 4.5, and then a powder mixture of sodium azide (0.65 g, 9.82 mmol, 1.5 equivalents) and sodium persulfate (2.36 g, 9.91 mmol, 1.5 equivalents) was added over 1.5 hours while maintaining the pH at 4 to 4.5.

After completion of dropping, the reaction liquid was stirred for 4 hours while maintaining the temperature and the pH. The reaction liquid was cooled to room temperature, sodium thiosulfate was added until the color of the iodine starch paper disappeared, and then the pH was adjusted to 0.5 with concentrated hydrochloric acid. The organic layer was separated with a separating funnel. As a result of HPLC analysis of the organic layer, it was confirmed that the organic layer contained methyl 3-azidoanthranilate (1.07 g, 84%) .

### [Comparative Example 1] (Synthesis in which tert-butyl hydroperoxide (TBHP) is used instead of persulfate)

To copper (I) bromide (0.0473 g, 0.33 mmol), acetonitrile (20 mL) was added to dissolve the copper (I) bromide at 20°C. To this solution, methyl anthranilate (0.5 g, 3.30 mmol) was added, and the mixture was stirred at 20°C for 10 minutes. Trimethylsilyl azide (0.762 g, 6.61 mmol) was added thereto, the mixture was stirred for 10 minutes, then tert-butyl hydroperoxide (0.595 g, 6.60 mmol) was added, and the mixture was stirred for 17 hours.

After completion of the reaction, the reaction liquid was cooled to 15°C or less, sodium thiosulfate was added until the color of the iodine starch paper disappeared, and then the pH of the reaction liquid was adjusted to 0.5 with concentrated hydrochloric acid. HPLC analysis of the organic layer and the aqueous layer confirmed that the reaction liquid contained no methyl 3-azidoanthranilate.

### [Comparative Example 2] (Synthesis in which hydrogen peroxide is used instead of persulfate)

To copper (II) acetate monohydrate (0.066 g, 0.33 mmol), water (10 mL) was added to dissolve the copper (II) acetate monohydrate at 20°C. To this solution, acetonitrile (20 mL) and methyl anthranilate (0.5 g, 3.30 mmol) were added, and the mixture was stirred at 20°C for 10 minutes. Sodium azide (0.43 g, 6.61 mmol) was added thereto, the mixture was stirred for 10 minutes, then a 30% hydrogen peroxide solution (0.749 g, 6.61 mmol) was added, and the mixture was stirred for 17 hours.

After completion of the reaction, the reaction liquid was cooled to 15°C or less, sodium thiosulfate was added until the color of the iodine starch paper disappeared, and then the pH of the reaction liquid was adjusted to 0.5 with concentrated hydrochloric acid. HPLC analysis of the organic layer and the aqueous layer confirmed that the reaction liquid contained methyl 3-azidoanthranilate (6.34 mg, 1.0%).

### [Examples 16 to 25] (Synthesis of azido form in which various aniline derivatives are used)

Synthesis of an azido form of an aniline derivative according to the following reaction formula was performed in the same manner as in Example 15 except that the raw material compound shown in Tables 1 to 3 below was used as an aniline derivative, and the reaction temperature and reaction time shown in the same Tables were applied. In Formula, SPS indicates sodium persulfate. The azido form of an aniline derivative, the obtained product, the yield and melting point, and the measurement data are shown in Tables 1 to 3.

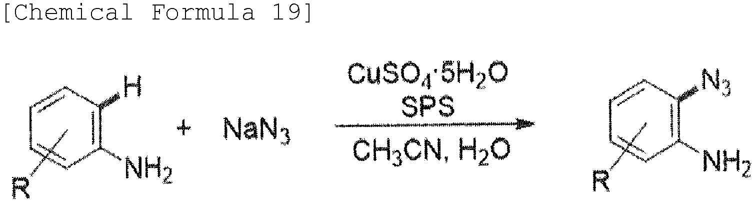

**[Table 1]**

| Example | Raw material | Product | Yield (%) | Reaction temperature (°C) | Reaction time (hour) | Melting point (°C) | IR(KBR) *v* (cm⁻¹) | ¹H-NMR(CDCl₃) *δ* (ppm) | ¹³C-NMR(CDCl₃) *δ* (ppm) |
|---|---|---|---|---|---|---|---|---|---|
| 16 | 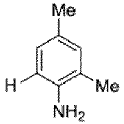 | 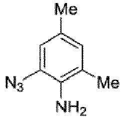 | 60 | 40 | 1 | 35∼35.5 | 2101 | 6.72 (1H, s), 6.68 (1H, s), 3.61 (2H, brs), 2.24 (3H, s), 2.12 (3H, s) | 133.8, 128.0, 127.6, 124.7, 123.6, 116.3, 20.5, 17.3 |
| 17 | 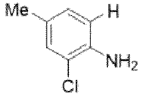 | 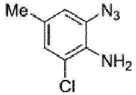 | 61 | 40 | 1.5 | 40∼41 | 2103 | 6.87 (1H, s), 6.75 (1H, s), 4.02 (2H, brs), 2.43 (3H, s) | 132.8, 128.2, 125.9, 119.6, 117.2, 20.4 |
| 18 | 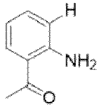 | 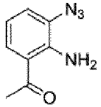 | 87 | 40 | 5 | 88∼89 | 2101, 1645 | 7.53 (1H, dd, J=8.0, 1.2 Hz), 7.14 (1H, dd, J=8.0, 1.6 Hz), 6.67 (1H, t, J=8.0 Hz), 6.55 (2H, brs), 2.57 (3H, s) | 200.4, 142.4, 128.2, 126.2, 122.0, 118.5, 114.9, 28.1 |
| 19 | 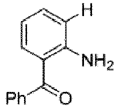 | 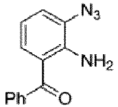 | 79 | 40 | 2.5 | 67-68 | 2107, 1635 | 7.64-7.61 (2H, m), 7.55-7.51 (1H, m), 7.47-7.43 (2H, m), 7.27 (1H, dd, J=8.0, 1.2 Hz), 7.17 (1H, dd, J=7.6, 1.2 Hz), 6.63 (1H, t, J=7 6 Hz), 6.32 (2H, brs) | 198.6, 142.9, 139.8, 131.3, 130.8, 129.2, 128.1, 126.2, 121.9, 118.6, 114.8 |

**[Table 2]**

| Example | Raw material | Product | Yield (%) | Reaction temperature (°C) | Reaction time (hour) | Melting point (°C) | IR(KBr) *v* (cm⁻¹) | ¹H-NMR(CDCl₃) *δ* (ppm) | ¹³C-NMR(CDCl₃) *δ* (ppm) |
|---|---|---|---|---|---|---|---|---|---|
| 20 | 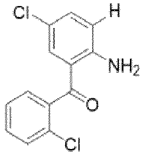 | 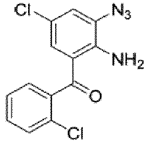 | 66 | 40 | 6 | 63-64 | 2105, 1635 | 7.46 (1H, dd, J=8.0, 1.2 Hz), 7,42 (1H, ddd, J=7.6, 7.2, 2.0 Hz), 7.37 (1H, ddd, 7.2, 7.2, 1.6 Hz), 7.29 (1H, dd, J=7.2, 1.2 Hz), 7.12 (1H, d, J=2.4 Hz), 6.94 (1H, d, J=2.4 Hz), 6.70 (2H, brs) | 196.1, 142.1, 138.8, 130.9, 130.7, 130.1, 129.3, 128.4, 127.6, 126.8, 122.7, 119.3, 118.0 |
| 21 | 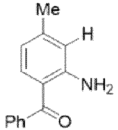 | 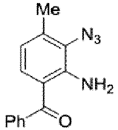 | 66 | 40 | 5 | <25 | 2098, 1606 | 7.61-7.59 (2H, m), 7.54-7.50 (1H, m), 7.46-7.42 (2H, m), 7.23 (1H, d, J=8.4 Hz), 6.52 (2H, brs), 6.41 (1H, d, J=8.4 Hz), 2.43 (3H, s) | 198.3, 145.0, 139.9, 138.2, 131.9, 131.1, 129.0, 128.1, 124.4, 117.4, 116.9, 18.2 |
| 22 | 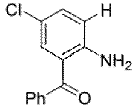 | 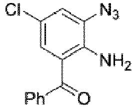 | 74 | 40 | 7 | 100∼104 | 2122, 1635 | 7.64-7.61 (2H, m), 7.58-7.54 (1H, m), 7.50-7.47 (2H, m), 7.23 (1H, d, J=2.4 Hz), 7.13 (1H, d, J=2.4 Hz), 6.29 (2H, brs) | 197.6, 141.6, 139.1, 131.8, 129.5, 129.2, 128.4, 127.6, 121.9, 119.3, 119.0 |

**[Table 3]**

| Example | Raw material | Product | Yield (%) | Reaction temperature (°C) | Reaction time (hour) | Melting point (°C) | IR(KBr) *v* (cm⁻¹) | ¹H-NMR(CDCl₃) *δ* (ppm) | ¹³C-NMR(CDCl₃) *δ* (ppm) |
|---|---|---|---|---|---|---|---|---|---|
| 23 | 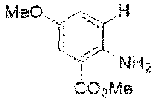 | 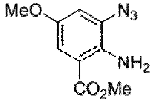 | 59 | 23 | 3 | 64∼65 | 2124, 1689 | 7.18 (1H, d, J=2.4 Hz), 6.83 (1H, d, J= 2.8 Hz), 5.64 (2H, brs), 3.88 (3H, s), 3.78 (3H, s) | 167.9, 149.9, 137.5, 111.6, 111.2, 109.7, 56.0, 51.8 |
| 24 | 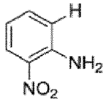 | 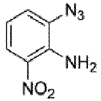 | 48 | 50 | 7 | 94∼95 | 2122, 1615 | 7.94 (1H, dd, J=8,8, 1.2 Hz), 7.23 (1H, dd, J=7.2, 1.2 Hz), 6.72 (1H, dd, J=8.8, 8.0 Hz), 6.33 (2H, brs) | 137.4, 128.1, 122.9, 122.3, 115.5 |
| 25 | 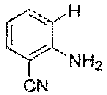 | 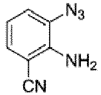 | 55 | 40 | 4 | 127.5∼128 | 2218, 2114 | 7.20-7.16 (2H, m), 6.76 (1H, t, J=8.0 Hz), 4.60 (2H, brs) | 141.7, 128.3, 125.9, 122.1, 117.9, 116.8, 96.7 |

### [Example 26] (Production of diaminobenzoic acid ester from azido form of anthranilic acid ester using zinc dust as reducing agent)

The reaction of Formula below was performed using zinc dust as a reducing agent.

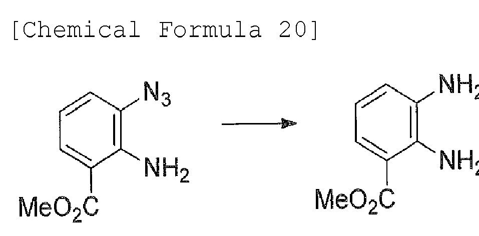

To methyl 3-azidoanthranilate obtained in Example 1 (10 mg, 0.05 mmol) and ammonium chloride (7 mg, 0.13 mmol), water (0.3 mL) and ethanol (1 mL) were added to dissolve them. Zinc dust (5 mg, 0.07 mmol) was added to this solution, and the mixture was stirred at room temperature for 16 hours. However, methyl 3-azidoanthranilate, a starting material, remained, thus, ammonium chloride (7 mg, 0.13 mmol) and zinc dust (5 mg, 0.07 mmol) were further added thereto, and the mixture was stirred at room temperature for 4 hours. The reaction liquid was diluted with ethyl acetate, then filtered, and the collected solid was washed with ethyl acetate. The filtrate and the washing were mixed, washed with a saturated saline, then dehydrated and filtered on magnesium sulfate, and the filtrate was analyzed by HPLC. The conversion rate from methyl 3-azidoanthranilate to methyl 2,3-diaminobenzoate was 99.7%.

The filtrate was concentrated under reduced pressure and then purified by a silica gel column (hexane/ethyl acetate = 1 : 1) to obtain methyl 2,3-diaminobenzoate (the yield determined by mass: 99%, the conversion rate from methyl 3-azidoanthranilate to methyl 2,3-diaminobenzoate determined by high performance liquid chromatography (HPLC): 99.7%, the purity by HPLC: 95%). The analytical values of the obtained methyl 2,3-diaminobenzoate were as follows.
IR(KBr) : 1693 cm⁻¹
1H-NMR (CDCl₃): δ 7.30 to 7.80 (m, 1H), 6.40 to 7.10 (m, 2H), 1.45 (brs, 2H), 3.85 (s, 3H), 3.40 (brs, 2H) [Example 27] (Production of diaminobenzoic acid ester from azido form of anthranilic acid ester using triphenylphosphine as reducing agent)

The reaction of Formula below was performed using triphenylphosphine (PPh₃) as a reducing agent.

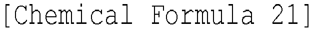

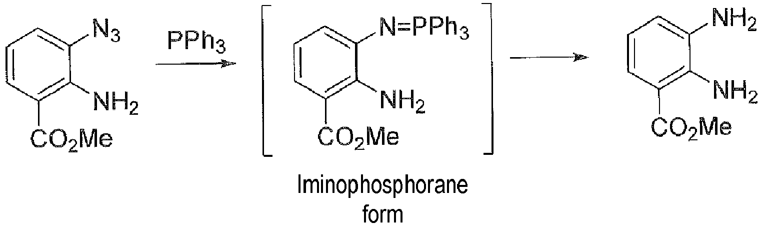

To a THF (0.5 mL) solution of methyl 3-azidoanthranilate (10 mg, 0.05 mmol) obtained in Example 1, triphenylphosphine (16 mg, 0.06 mmol) and water (0.01 g) were added, and the mixture was stirred and reacted at 55°C for 16 hours. The reaction liquid was concentrated under reduced pressure until THF and water stopped volatiling, water (1 mL) and ethyl acetate (1 mL) were added, and then hydrochloric acid was added to adjust the pH to 1 and hydrolyze the mixture. The pH of the aqueous layer obtained by liquid separation was adjusted to 10 with 24% NaOH, and the organic layer was concentrated under reduced pressure after extraction with ethyl acetate to obtain methyl 2,3-diaminobenzoate.

Purified methyl 2,3-diaminobenzoate was obtained in the same manner as in Example 26 (the yield determined by mass: 95%, the conversion rate from methyl 3-azidoanthranilate to methyl 2,3-diaminobenzoate determined by high performance liquid chromatography (HPLC): 99%, the purity by HPLC: 95%).

### [Example 28] (Production of benzimidazole derivative from diaminobenzoic acid ester)

The reaction of Formula below was performed using an ortho ester derivative.

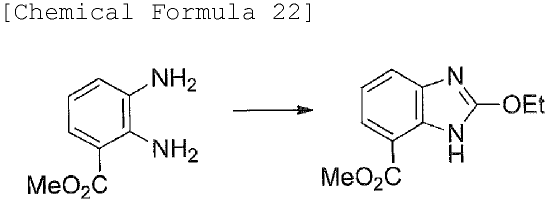

Methyl 2,3-diaminobenzoate (10 mg, 0.06 mmol diaminobenzoic acid ester) obtained in Example 26 was dissolved in toluene (1 mL), acetic acid (4 mg, 0.07 mmol) and tetraethoxymethane (10 mg, 0.08 mmol; ortho ester derivative) were added at room temperature, and the mixture was stirred and reacted at 100°C for 2 hours. HPLC analysis of the reaction liquid confirmed the conversion rate from methyl 2,3-diaminobenzoate to the benzimidazole derivative to be 100%.

The reaction liquid was crystallized by addition of water (0.5 mL) and filtered to obtain methyl 2-ethoxy-1H-benzimidazole-7-carboxylate (11.9 mg, yield: 90%). The purity of methyl 2-ethoxy-1H-benzimidazole-7-carboxylate confirmed by HPLC was 95%.

## Claims

1. A method for producing an α-azidoaniline derivative or an α,α'-diazide derivative represented by Formula (2) below: 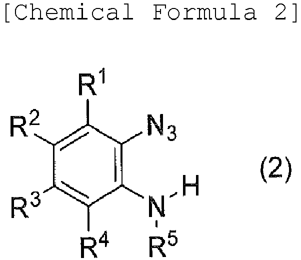 (wherein R¹ to R³, and R⁵ are each independently a hydrogen atom, a halogen atom, a nitro group, a cyano group, a substituted or unsubstituted alkyl group having 1 to 12 carbon atoms, a substituted or unsubstituted alkoxy group having 1 to 12 carbon atoms, a substituted or unsubstituted aralkyl group having 7 to 14 carbon atoms, a substituted or unsubstituted aryl group having 2 to 13 carbon atoms, a substituted or unsubstituted alkoxycarbonyl group having 2 to 7 carbon atoms, a substituted or unsubstituted carbonyl group having 1 to 20 carbon atoms, or optionally form an aliphatic hydrocarbon ring, an aromatic ring, or a heterocyclic ring together with an adjacent group, and R⁴ is an azido group (N₃), a hydrogen atom, a halogen atom, a nitro group, a cyano group, a substituted or unsubstituted alkyl group having 1 to 12 carbon atoms, a substituted or unsubstituted alkoxy group having 1 to 12 carbon atoms, a substituted or unsubstituted aralkyl group having 7 to 14 carbon atoms, a substituted or unsubstituted aryl group having 2 to 13 carbon atoms, a substituted or unsubstituted alkoxycarbonyl group having 2 to 7 carbon atoms, a substituted or unsubstituted carbonyl group having 1 to 20 carbon atoms, or optionally forms an aliphatic hydrocarbon ring, an aromatic ring, or a heterocyclic ring together with an adjacent group),
comprising the step of:
contacting an aniline derivative represented by Formula (1) below: 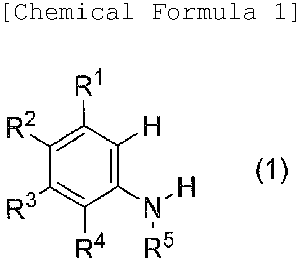 (wherein R¹ to R⁵ are each independently a hydrogen atom, a halogen atom, a nitro group, a cyano group, a substituted or unsubstituted alkyl group having 1 to 12 carbon atoms, a substituted or unsubstituted alkoxy group having 1 to 12 carbon atoms, a substituted or unsubstituted aralkyl group having 7 to 14 carbon atoms, a substituted or unsubstituted aryl group having 2 to 13 carbon atoms, a substituted or unsubstituted alkoxycarbonyl group having 2 to 7 carbon atoms, a substituted or unsubstituted carbonyl group having 1 to 20 carbon atoms, or optionally form an aliphatic hydrocarbon ring, an aromatic ring, or a heterocyclic ring together with an adjacent group)
with an azidating agent in presence of water, a persulfate, and a copper compound.

2. The method according to claim 1, wherein the aniline derivative represented by Formula (1) above is an anthranilic acid ester represented by Formula (3) below: 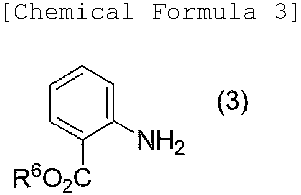 (wherein R⁶ is an alkyl group having 1 to 6 carbon atoms, an alkoxyalkyl group having 2 to 12 carbon atoms, an aralkyl group having 7 to 10 carbon atoms, or an alkoxyaralkyl group having 8 to 11 carbon atoms).

3. The method according to claim 1 or 2, wherein the azidating agent is at least one selected from sodium azide, tetra-n-butylammonium azide, tetramethylammonium azide, tetraethylammonium azide, and benzyltriethylammonium azide.

4. The method according to any one of claims 1 to 3, wherein the persulfate is at least one selected from sodium persulfate, potassium persulfate, ammonium persulfate, and potassium hydrogen persulfate.

5. The method according to any one of claims 1 to 4, wherein a reaction liquid when the aniline derivative is contacted with the azidating agent has a pH of 3.0 to 7.0.

6. The method according to any one of claims 1 to 5, wherein a reaction solvent used when the aniline derivative is contacted with the azidating agent is water or a water-containing solvent.

7. A method for producing a diaminobenzoic acid ester represented by Formula (5) below: 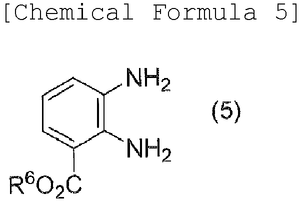 (wherein R⁶ is an alkyl group having 1 to 6 carbon atoms, an alkoxyalkyl group having 2 to 12 carbon atoms, an aralkyl group having 7 to 10 carbon atoms, or an alkoxyaralkyl group having 8 to 11 carbon atoms),
comprising the step of:
reducing an azido form of an anthranilic acid ester represented by Formula (4) below: 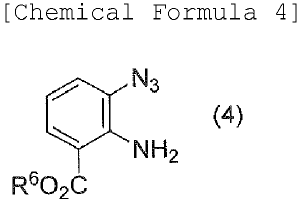 (wherein R⁶ has a meaning same as that of R⁶ in Formula (5) above).

8. The method for producing a diaminobenzoic acid ester according to claim 7, comprising the step of:
producing an azido form of an anthranilic acid ester represented by Formula (4) above by the method according to claim 2.

9. A method for producing a benzimidazole derivative represented by Formula (7) below: 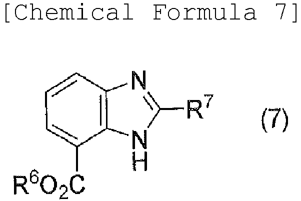 (wherein R⁶ has a meaning same as that of R⁶ in Formula (3) above, and R⁷ is an alkyl group having 1 to 6 carbon atoms or an alkoxy group having 1 to 6 carbon atoms),
comprising the step of:
producing a diaminobenzoic acid ester represented by Formula (5) above by the method according to claim 7 or 8, and then contacting the obtained diaminobenzoic acid ester with an ortho ester derivative represented by Formula (6) below: 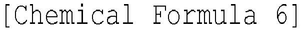 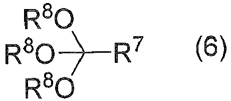 (wherein R⁷ has a meaning same as that of R⁷ in Formula (7) above, and R⁸ is an alkyl group having 1 to 6 carbon atoms, and is optionally same or different each other)
in presence of an acid.

10. An azido form of an anthranilic acid ester represented by Formula (4) below: 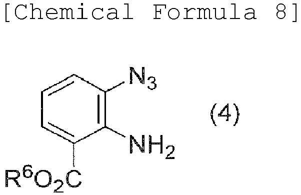 (wherein R⁶ is an alkyl group having 1 to 6 carbon atoms, an alkoxyalkyl group having 2 to 12 carbon atoms, an aralkyl group having 7 to 10 carbon atoms, or an alkoxyaralkyl group having 8 to 11 carbon atoms).
